# EUROPEAN PATENT SPECIFICATION

(11) **EP 3 934 559 B1**
(45) Date of publication and mention of the grant of the patent: **27.08.2025**
(21) Application number: 20770962.7
(22) Date of filing: 06.03.2020
(51) Int. Cl.: A61B 34/30, A61B 34/00, A61B 90/00, A61B 90/30, A61B 34/20, A61B 17/00

(54) **ROBOTIC SURGERY SYSTEMS**
SYSTEM FÜR ROBOTISCHE CHIRURGIE
SYSTÈMES CHIRURGICAUX ROBOTIQUES

(30) Priority: 08.03.2019 US 201962815420 P
(43) Date of publication of application: 12.01.2022
(73) Proprietor: Covidien LP, Mansfield, MA 02048 (US)
(72) Inventor: MAZZARO, Luciano, Boulder, Colorado 80304 (US)
(74) Representative: Maschio & Soames IP Ltd
(86) International application number: PCT/US2020/021471
(87) International publication number: WO 2020/185589

(56) References cited:
- WO-A1-2018/118390
- WO-A1-2018/217433
- WO-A2-2018/217444
- US-A1- 2009 088 634
- US-A1- 2017 189 127
- US-A1- 2018 168 733
- US-A1- 2018 168 734
- US-A1- 2018 262 656
- US-A1- 2019 047 151
- US-B2- 9 949 800

## Description

### BACKGROUND

Iatrogenic injuries are unintended injuries caused by surgical instruments during a surgical procedure. While surgery often requires cutting, puncturing, ablation, and/or removal of tissue, not all such cutting, puncturing, ablation, and/or removal of tissue occurring during a surgical procedure are always intentional or therapeutic. In some instances, iatrogenic injuries may be severe, such as by accidentally cutting a major blood vessel or puncturing an organ. Thus, iatrogenic injuries should be avoided as much as possible. In open surgical settings, a clinician often has a clearer field of view than during laparoscopic surgical procedures that limit the clinician's view of a surgical site to images captured by one or more laparoscopes placed about the surgical site. While iatrogenic injuries may occur during any surgical procedure, such injuries are particularly prevalent in laparoscopic procedures during which the clinician's view of the surgical site, and in particular of the surgical tools, is limited. Relevant prior art can be found in US 9 949 800 B2, WO 2018/118390 A1, US 2017/189127 A1, US 2009/088634 A1 and WO 2018/217433 A1 Disclosed hereinbelow are systems, apparatus, and methods for reducing iatrogenic injuries during surgical procedures involving a surgical robot.

### SUMMARY

The invention is defined by the appended claims 1 and 9. Methods disclosed herein do not form part of the claimed subject-matter. The present disclosure relates to robotic surgery, and more particularly, to systems, apparatus, and methods for limiting movement of surgical tools and/or robotic arms outside a laparoscopic field of view.

Provided in accordance with the present disclosure is a system for performing robotic surgery. In an aspect of the present invention, the system includes a surgical robot including a plurality of robotic arms, an image capture device and a light source both operably coupled to a first one of the plurality of robotic arms a surgical tool operably coupled to a second one of the plurality of robotic arms and a light sensor located at the distal end of said tool, and a computing device including a processor and a memory storing instructions which, when executed by the processor, cause the computing device to determine whether the light sensor coupled to the surgical tool is detecting at least a predetermined amount of light emitted by the light source coupled to the image capture device, and provide a notification when it is determined that the light sensor coupled to the surgical tool is not detecting at least the predetermined amount of light emitted by the light source coupled to the image capture device.

In another aspect of the present disclosure, the instructions of the computing device, when executed by the processor, further cause the computing device to cause the surgical robot to reposition the surgical tool when it is determined that the light sensor coupled to the surgical tool is not detecting at least the predetermined amount of light emitted by the light source coupled to the image capture device.

In a further aspect of the present disclosure, the surgical tool is repositioned towards the image capture device until the light sensor detects at least the predetermined amount of light emitted by the light source coupled to the image capture device.

In another aspect of the present disclosure, the surgical tool is a first surgical tool and the light sensor is a first light sensor, and the system further includes a second surgical tool coupled to a third one of the plurality of robotic arms and a second light sensor, and the instructions, when executed by the processor, further cause the computing device to determine whether the second light sensor coupled to the second surgical tool is detecting at least a second predetermined amount of light emitted by the light source coupled to the image capture device, and provide a notification when it is determined that the second light sensor coupled to the second surgical tool is not detecting at least the second predetermined amount of light emitted by the light source coupled to the image capture device.

In a further aspect of the present disclosure, the instructions, when executed by the processor, further cause the computing device to cause the surgical robot to reposition the second surgical tool when it is determined that the second light sensor coupled to the second surgical tool is not detecting at least the second predetermined amount of light emitted by the light source coupled to the image capture device.

In yet another aspect of the present disclosure, the system further includes a door for the light sensor coupled to the surgical tool, and the instructions, when executed by the processor, further cause the computing device to determine whether the surgical tool is inserted into a trocar, cause the surgical robot to open the door for the light sensor when it is determined that the surgical tool is inserted into the trocar, and cause the surgical robot to close the door for the light sensor when it is determined that the surgical tool is not inserted into the trocar.

In still another aspect of the present disclosure, the instructions, when executed by the processor, further cause the computing device to prevent activation of the surgical tool when it is determined that the light sensor coupled to the surgical tool is not detecting at least the predetermined amount of light emitted by the light source coupled to the image capture device.

In yet another aspect of the present disclosure, the system further includes a pump for circulating coolant through the surgical tool, and the instructions further cause the computing device to activate the pump to circulate coolant through the surgical tool when it is determined that the light sensor coupled to the surgical tool is not detecting at least the predetermined amount of light emitted by the light source coupled to the image capture device.

In still another aspect of the present disclosure, the surgical tool is an electrosurgical tool, and the system further includes an electrosurgical generator configured to provide electrosurgical energy to the surgical tool, and the instructions, when executed by the processor, further cause the computing device to prevent the electrosurgical generator from providing electrosurgical energy to the surgical tool when it is determined that the light sensor coupled to the surgical tool is not detecting at least the predetermined amount of light emitted by the light source coupled to the image capture device.

In yet another aspect of the present disclosure, the image capture device is a laparoscope.

In still another aspect of the present disclosure, the predetermined amount of light is less than a total amount of light emitted by the light source coupled to the image capture device.

Provided in accordance with the present disclosure is a method for robotic surgery. In an aspect of the present disclosure, the method includes determining whether a light sensor coupled to a surgical tool is detecting at least a predetermined amount of light emitted by a light source coupled to an image capture device, and providing a notification when it is determined that the light sensor coupled to the surgical tool is not detecting at least the predetermined amount of light emitted by the light source coupled to the image capture device.

In another aspect of the present disclosure, the surgical tool is coupled to a robotic arm of a surgical robot, and the method further includes causing the surgical robot to reposition the surgical tool when it is determined that the light sensor coupled to the surgical tool is not detecting at least the predetermined amount of light emitted by the light source coupled to the image capture device.

In a further aspect of the present disclosure, the surgical tool is repositioned towards the image capture device until the light sensor detects at least the predetermined amount of light emitted by the light source coupled to the image capture device.

In another aspect of the present disclosure, the surgical tool is a first surgical tool and the light sensor is a first light sensor, and the method further includes determining whether a second light sensor coupled to a second surgical tool is detecting at least a second predetermined amount of light emitted by the light source coupled to the image capture device, and providing a notification when it is determined that the second light sensor coupled to the second surgical tool is not detecting at least the second predetermined amount of light emitted by the light source coupled to the image capture device.

In a further aspect of the present disclosure, the second surgical tool is coupled to a robotic arm of a surgical robot, and the method further comprises causing the surgical robot to reposition the second surgical tool when it is determined that the second light sensor coupled to the second surgical tool is not detecting at least the second predetermined amount of light emitted by the light source coupled to the image capture device.

In another aspect of the present disclosure, the method further includes determining whether the surgical tool is inserted into a trocar, opening a door for the light sensor when it is determined that the surgical tool is inserted into the trocar, and closing the door for the light sensor when it is determined that the surgical tool is not inserted into the trocar.

In yet another aspect of the present disclosure, the method further includes preventing activation of the surgical tool when it is determined that the light sensor coupled to the surgical tool is not detecting at least the predetermined amount of light emitted by the light source coupled to the image capture device.

In still another aspect of the present disclosure, the method further includes activating a pump to circulate coolant through the surgical tool when it is determined that the light sensor coupled to the surgical tool is not detecting at least the predetermined amount of light emitted by the light source coupled to the image capture device.

In yet another aspect of the present disclosure, the surgical tool is an electrosurgical tool, and the method further comprises preventing an electrosurgical generator from providing electrosurgical energy to the surgical tool when it is determined that the light sensor coupled to the surgical tool is not detecting at least the predetermined amount of light emitted by the light source coupled to the image capture device.

In still another aspect of the present disclosure, the predetermined amount of light is less than a total amount of light emitted by the light source coupled to the image capture device.

Provided in accordance with an aspect of the present invention is a non-transitory computer-readable storage medium storing instructions which, when executed by a processor in the system for robotic surgery mentioned above, cause a computer to determine whether a light sensor coupled to a surgical tool is detecting at least a predetermined amount of light emitted by a light source coupled to an image capture device, and providing a notification when it is determined that the light sensor coupled to the surgical tool is not detecting at least the predetermined amount of light emitted by the light source coupled to the image capture device.

In another aspect of the present disclosure, the surgical tool is coupled to a robotic arm of a surgical robot, and the instructions, when executed by the processor, further cause the computer to cause the surgical robot to reposition the surgical tool when it is determined that the light sensor coupled to the surgical tool is not detecting at least the predetermined amount of light emitted by the light source coupled to the image capture device.

In a further aspect of the present disclosure, the surgical tool is repositioned towards the image capture device until the light sensor detects at least the predetermined amount of light emitted by the light source coupled to the image capture device.

In another aspect of the present disclosure, the surgical tool is a first surgical tool and the light sensor is a first light sensor, and the instructions, when executed by the processor, further cause the computer to determine whether a second light sensor coupled to a second surgical tool is detecting at least a second predetermined amount of light emitted by the light source coupled to the image capture device, and provide a notification when it is determined that the second light sensor coupled to the second surgical tool is not detecting at least the second predetermined amount of light emitted by the light source coupled to the image capture device.

In a further aspect of the present disclosure, the second surgical tool is coupled to a robotic arm of a surgical robot, and the instructions, when executed by the processor, further cause the computer to cause the surgical robot to reposition the second surgical tool when it is determined that the second light sensor coupled to the second surgical tool is not detecting at least the second predetermined amount of light emitted by the light source coupled to the image capture device.

In another aspect of the present disclosure, the instructions, when executed by the processor, further cause the computer to determine whether the surgical tool is inserted into a trocar, open a door for the light sensor when it is determined that the surgical tool is inserted into the trocar, and close the door for the light sensor when it is determined that the surgical tool is not inserted into the trocar.

In yet another aspect of the present disclosure, the instructions, when executed by the processor, further cause the computer to prevent activation of the surgical tool when it is determined that the light sensor coupled to the surgical tool is not detecting at least the predetermined amount of light emitted by the light source coupled to the image capture device.

In still another aspect of the present disclosure, the instructions, when executed by the processor, further cause the computer to activate a pump to circulate coolant through the surgical tool when it is determined that the light sensor coupled to the surgical tool is not detecting at least the predetermined amount of light emitted by the light source coupled to the image capture device.

In yet another aspect of the present disclosure, the surgical tool is an electrosurgical tool, and the instructions, when executed by the processor, further cause the computer to prevent an electrosurgical generator from providing electrosurgical energy to the surgical tool when it is determined that the light sensor coupled to the surgical tool is not detecting at least the predetermined amount of light emitted by the light source coupled to the image capture device.

In still another aspect of the present disclosure, the predetermined amount of light is less than a total amount of light emitted by the light source coupled to the image capture device.

Any of the above aspects and embodiments of the present disclosure may be combined without departing from the scope of the present disclosure.

### BRIEF DESCRIPTION OF THE DRAWINGS

Various aspects and features of the present disclosure are described hereinbelow with references to the drawings, wherein:
FIG. 1 is a schematic diagram of an exemplary system for robotic surgery according to an embodiment of the present disclosure;
FIG. 2 is a box diagram of an exemplary computing device forming part of the system of FIG. 1, according to an embodiment of the present disclosure; and
FIG. 3 is a flowchart of an exemplary method for robotic surgery according to an embodiment of the present disclosure.

### DETAILED DESCRIPTION

The present disclosure relates to systems, apparatus, and methods for performing robotic surgery. More particularly, the disclosed systems, apparatus, and tracks surgical tools leaving a field of view of an imaging device, such as a laparoscopic camera, and thus a clinician's view of a surgical site, during robotic surgery. Surgical tools may be moved, whether intentionally or inadvertently, about the surgical site during a robotic surgical procedure. The clinician's view of the surgical site may also be changed due to movement of the imaging device. Thus, as a result of either or both of the movement of the surgical tools and/or the imaging device, the surgical tools may leave the clinician's view of the surgical site.

Robotic surgical systems in accordance with the present disclosure track the location of the surgical tools within the clinician's view of the surgical site and prevents movement and/or activation of the surgical tools outside of the clinician's view.

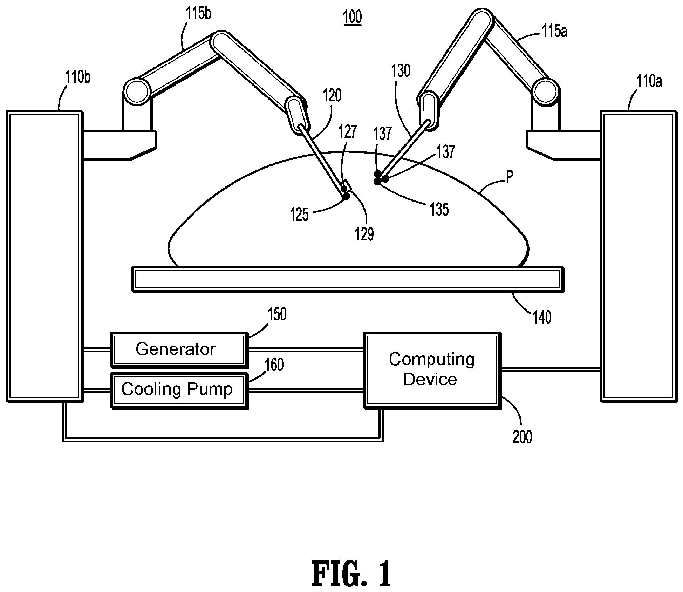

With reference to FIG. 1, there is shown a schematic diagram of an exemplary system 100 for use during a robotic surgical procedure according to an embodiment of the present disclosure. System 100 includes a surgical robot having a plurality of robotic bases 110a, 110b. Each robotic base 110a, 110b has operatively coupled thereto one or more robotic arms 115a, 115b. Each robotic arm 115a, 115b has in turn operatively coupled thereto a tool, such as a surgical tool 120 and/or an image capture device 130.

Surgical tool 120 may be a cutting tool, ablation tool, forceps, graspers, stapling tools, suturing tools, etc. and may have an end effector 125 at or about a distal portion of surgical tool 120. Surgical tool 120 has coupled thereto a light sensor 127, such as a photocell or other sensor configured to detect light, coupled at or about the distal portion of surgical tool 120. Light sensor 127 may be protected from exposure to light when not in use by a protective cover or door 129.

Image capture device 130 may be a laparoscope or other device configured to capture images. Image capture device 130 may have a lens or camera 135 at or about a distal portion of image capture device. Image capture device 130 has coupled thereto a light source 137, such as a fiber optic light source or other cold light source configured to emit light.

System 100 further includes a table 140 upon which a patient "P" is positioned during surgery, a generator 150 configured to provide electrosurgical energy to surgical tool 120, a cooling pump 160 configured to pump fluid through surgical tool 120 to cool surgical tool 120, and a computing device 200.

As further described below, during a surgical procedure, computing device 200 may cause the surgical robot to insert surgical tool 120 and/or image capture device 130 into the patient's body via one or more trocars (not shown). Once inserted via the trocar, door 129 may be opened to allow light sensor 127 to detect light emitted by light source 137. During the surgical procedure, light source 137 will illuminate a surgical site inside the patient's body, and because light source 137 is coupled to image capture device 130, the area illuminated by the light emitted by light source 137 will roughly correspond with a field of view of the surgical site captured by image capture device 130, and thus the clinician's view of the surgical site. As such, during the surgical procedure, computing device 200, by means of processor 204, other logic, and/or an application 280, may continually monitor whether light sensor 127 detects light emitted by light source 137, and may allow or prevent activation of surgical tool 120 based on whether light sensor 127 detects light emitted by light source 137. Computing device 200 may also cause the surgical robot to reposition surgical tool 120 to be within the area illuminated by light source 137.

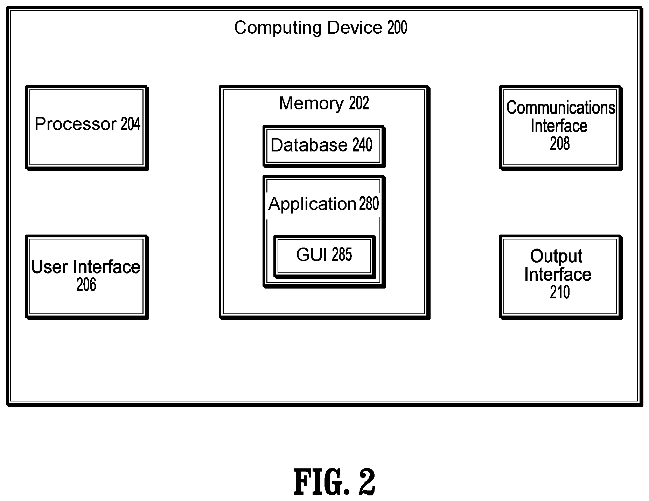

Turning now to FIG. 2, there is shown a schematic diagram of a computing device 200 forming part of system 100 of FIG. 1, according to an embodiment of the present disclosure. Computing device 200 includes a memory 202 storing a database 240 and an application 280. Application 280 may include instructions which, when executed by a processor 204, cause computing device 200 to perform various functions. Computing device 200 may further include a graphical user interface (GUI) 285. In some embodiments, GUI 285 may be part of and/or included in application 280. In other embodiments, GUI 285 may be separate from application 280, such as an operating system of computing device 200.

Memory 202 of computing device 200 may include any non-transitory computer-readable storage medium for storing data and/or software that is executable by processor 204 and which controls the operation of computing device 200. In an embodiment, memory 202 may include one or more solid-state storage devices such as flash memory chips. Alternatively or in addition to the one or more solid-state storage devices, memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media may include, but is not limited to, non-transitory, volatile and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by computing device 200.

Computing device 200 further includes a user interface 206, a communications interface 208, and an output interface 210. User interface 206 may be a mouse, keyboard, or other hand-held controller, foot pedal, touch screen, voice interface, and/or any other device or interface by means of which a user may interact with computing device 200.

Communications interface 208 may be configured to connect to a network such as a local area network (LAN) consisting of a wired network and/or a wireless network, a wide area network (WAN), a wireless mobile network, a BLUETOOTH^{®} network, and/or the internet. Output interface 210 may be a screen or other display device usable to output images, data, and/or GUI 285 by computing device 200.

As noted above, application 280 includes instructions which, when executed by processor 204, control the operation of computing device 200. Various functions of computing device 200 are described below. Such functions may be performed by dedicated hardware or other logic included in computing device 200, and/or by execution of application 280. Thus, those skilled in the art will recognize that some or all of the functions and operations of computing device 200 described herein may be performed by hardware, software, and/or firmware included in and/or executed by computing device 200.

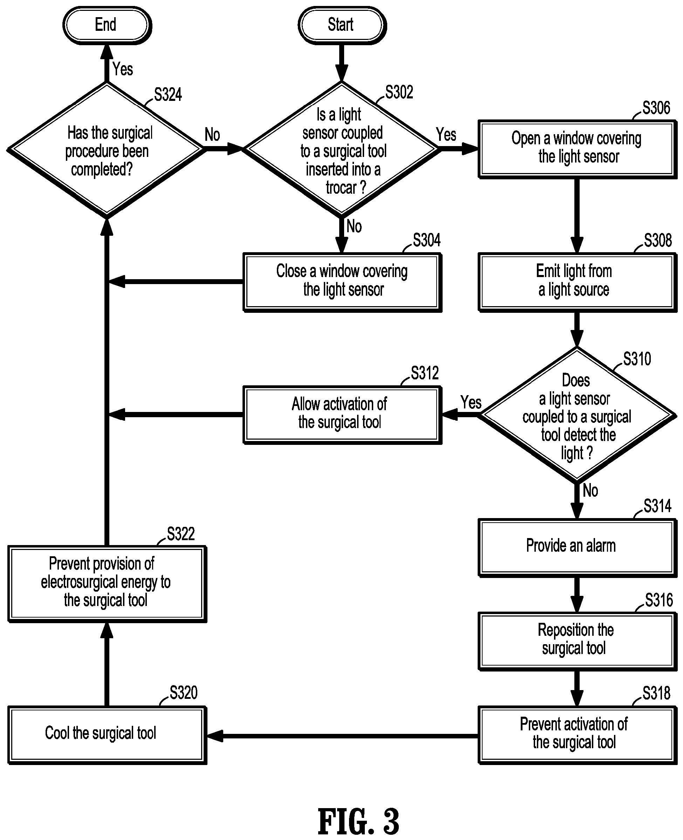

With reference to FIG. 3, there is shown a flowchart of an exemplary method 300 for robotic surgery according to embodiments of the present disclosure. Method 300 may be performed, for example, by using application 280 executing on computing device 200 of FIG. 2 and system 100 of FIG. 1. While method 300 is described below in an ordered sequence of steps, those skilled in the art will recognize that one or more of the steps may be performed out of sequence, in a different sequence, repeated, and/or omitted without departing from the scope of the present disclosure.

Method 300 may start at step S302, where it is determined whether light sensor 127, coupled to surgical tool 120, is inserted via a trocar into the patient's body. The determination may be made by application 280 executing on computing device 200, and/or specialized or dedicated logic coupled to light sensor 127, surgical tool 120, and/or the trocar. If it is determined that light sensor 127 is not inserted via the trocar, processing proceeds to step S304 where door 129 is closed, or kept closed, to cover light sensor 127. Thereafter, processing proceeds to step S324, described below.

Alternatively, if it is determined at step S302 that light sensor 127 is inserted via the trocar, processing proceeds to step S306, where door 129 is opened, or kept open, to uncover light sensor 127. Thereafter, processing proceeds to step S308, where light source 137 is caused to emit light. For example, once application 280 determines or is notified that light sensor 127 is uncovered, application 280 may cause light source 137 to emit light. At this point, or at some point prior, surgical tool 120 and image capture device 130 are positioned about a surgical site inside the patient's body. Additional surgical tools 120 and image capture devices 130 may also be inserted via trocars and positioned about the surgical site inside the patient's body.

Thereafter, at step S310, application 280 determines whether light sensor 127 is detecting the light emitted by light source 137. For example, computing device 200 may receive a signal from light sensor 127, or from logic associated with light sensor 127, indicating that light sensor 127 is detecting light. If application 280 determines that light sensor 127 is detecting light, it may be assumed that surgical tool 120 is within the field of view of image capture device 130, and processing proceeds to step S312, where application 280 allows activation of surgical tool 120. For example, if surgical tool 120 is an ablation tool, application 280 may allow generator 150 to provide electrosurgical energy to surgical tool 120. Thereafter, processing proceeds to step S324, described below.

Alternatively, if application 280 determines that light sensor 127 is not detecting light, or that light sensor 127 is detecting insufficient light, (for example, by comparing an amount of light sensed by the light sensor 127 to a predetermined threshold and/or determining whether the light sensor 127 is detecting at least a predetermined amount of light emitted by the light source 137) it may be assumed that surgical tool 120 is not within the field of view of image capture device 130. For example, if light sensor 127 detects insufficient light, that is, light below the predetermined threshold, or no light at all, application 280 may determine that surgical tool 120 is not within the field of view of image capture device 130. Thereafter, processing proceeds to step S314, where application 280 provides an alert or alarm to the clinician indicating that surgical tool 120 is not within the field of view of image capture device 130. The alert or alarm may be an auditory, visual, or tactile alert to inform the clinician that surgical tool 120 is outside of the clinician's view of the surgical site.

Application 280 may further, at step S316, cause the surgical robot to reposition surgical tool 120 towards image capture device 130 until light sensor 127 detects light emitted by light source 137. The repositioning may be automatic or dependent on instruction by the clinician. Application 280 may also, at step S318, prevent activation of surgical tool 120 while light sensor 127 does not detect light emitted by light source 137. Additionally, application 280 may, at step S320, cause cooling pump 160 to pump cooling fluid through surgical tool 120 to cool surgical tool 120 while light sensor 127 does not detect light emitted by light source 137. Further, application 280 may, at step S322, stop and/or prevent generator 150 from providing electrosurgical energy to surgical tool 120. While the above-described steps S316-S322 are described in sequence, those skilled in the art will realize that one or more of such steps may be omitted depending on the type of surgical tool 120 being used and/or may be performed in a different order or sequence than described here, without departing from the scope of the present disclosure.

Thereafter, processing proceeds to step S324, where application 280 determines whether the surgical procedure has been completed. If application 280 determines that the surgical procedure has not been completed, processing returns to step S302, where application 280 determines whether light sensor 127 remains inserted via the trocar. Alternatively, if application 280 determines at step S324 that the surgical procedure has been completed, processing ends.

While the above-described steps of method 300 describe an embodiment where a surgical robot is used to perform the surgery, those skilled in the art will recognize that the same or similar steps may be performed by a clinician performed manual laparoscopic surgery. For example, all of steps S302-S324, with the exception of the automatic repositioning of surgical tool 120 at step S316, are also applicable to manual laparoscopic surgery and the same or similar devices as described herein may be used without the surgical robot.

Detailed embodiments of devices, systems incorporating such devices, and methods using the same as described herein. However, these detailed embodiments are merely examples of the disclosure, which may be embodied in various forms. Therefore, specific structural and functional details disclosed herein are not to be interpreted as limiting, but merely as a basis for the claims and as a representative basis for allowing one skilled in the art to variously employ the present disclosure in appropriately detailed structure.

## Claims

1. A system (100) for robotic surgery, the system comprising:
a surgical robot including a plurality of robotic arms (115a, 115b);
an image capture device (130) and a light source (137) both operably coupled to a first one of the plurality of robotic arms
a surgical tool (120) operably coupled to a second one of the plurality of robotic arms and a light sensor (127) located at the distal end of said tool; and
a computing device (200) including a processor (204) and a memory (202) storing instructions which,
when executed by the processor, cause the computing device to:
determine whether the light sensor coupled to the surgical tool is detecting at least a predetermined amount of light emitted by the light source coupled to the image capture device; and
provide a notification when it is determined that the light sensor coupled to the surgical tool is not detecting at least the predetermined amount of light emitted by the light source coupled to the image capture device.

2. The system according to claim 1, wherein the instructions of the computing device, when executed by the processor, further cause the computing device to cause the surgical robot to reposition the surgical tool when it is determined that the light sensor coupled to the surgical tool is not detecting at least the predetermined amount of light emitted by the light source coupled to the image capture device.

3. The system according to claim 2, wherein the surgical tool is repositioned towards the image capture device until the light sensor detects at least the predetermined amount of light emitted by the light source coupled to the image capture device.

4. The system according to claim 2, wherein the surgical tool is a first surgical tool and the light sensor is a first light sensor, the system further comprising:
a second surgical tool coupled to a third one of the plurality of robotic arms and a second light sensor,
wherein the instructions, when executed by the processor, further cause the computing device to:
determine whether the second light sensor coupled to the second surgical tool is detecting at least a second predetermined amount of light emitted by the light source coupled to the image capture device; and
provide a notification when it is determined that the second light sensor coupled to the second surgical tool is not detecting at least the second predetermined amount of light emitted by the light source coupled to the image capture device; preferably wherein the instructions, when executed by the processor, further cause the computing device to cause the surgical robot to reposition the second surgical tool when it is determined that the second light sensor coupled to the second surgical tool is not detecting at least the second predetermined amount of light emitted by the light source coupled to the image capture device.

5. The system according to any preceding claim, wherein the light sensor coupled to the surgical tool is located at a distal portion of the surgical tool; and/or further comprising a door for the light sensor coupled to the surgical tool,
wherein the instructions, when executed by the processor, further cause the computing device to:
determine whether the surgical tool is inserted into a trocar;
cause the surgical robot to open the door for the light sensor when it is determined that the surgical tool is inserted into the trocar; and
cause the surgical robot to close the door for the light sensor when it is determined that the surgical tool is not inserted into the trocar.

6. The system according to any preceding claim, wherein the instructions, when executed by the processor, further cause the computing device to prevent activation of the surgical tool when it is determined that the light sensor coupled to the surgical tool is not detecting at least the predetermined amount of light emitted by the light source coupled to the image capture device; and/or further comprising:
a pump for circulating coolant through the surgical tool,
wherein the instructions further cause the computing device to activate the pump to circulate coolant through the surgical tool when it is determined that the light sensor coupled to the surgical tool is not detecting at least the predetermined amount of light emitted by the light source coupled to the image capture device.

7. The system according to any preceding claim, wherein the surgical tool is an electrosurgical tool, and the system further comprises:
an electrosurgical generator configured to provide electrosurgical energy to the surgical tool,
wherein the instructions, when executed by the processor, further cause the computing device to prevent the electrosurgical generator from providing electrosurgical energy to the surgical tool when it is determined that the light sensor coupled to the surgical tool is not detecting at least the predetermined amount of light emitted by the light source coupled to the image capture device.

8. The system according to any preceding claim, wherein the image capture device is a laparoscope; and/or wherein the predetermined amount of light is less than a total amount of light emitted by the light source coupled to the image capture device.

9. A non-transitory computer-readable storage medium storing instructions which, when executed by a processor (204) in a system for robotic surgery, the system (100) comprising:
a surgical robot including a plurality of robotic arms (115, 115b);
an image capture device (130) and a light source (137) both operably coupled to a first one of the plurality of robotic arms and
a surgical tool (120) operably coupled to a second one of the plurality of robotic arms and a light sensor (127) located at the distal end of said tool;
cause a computer (200) to:
determine whether a light sensor coupled to the surgical tool is detecting at least a predetermined amount of light emitted by the light source coupled to an image capture device; and
provide a notification when it is determined that the light sensor coupled to the surgical tool is not detecting at least the predetermined amount of light emitted by the light source coupled to the image capture device.

10. The non-transitory computer-readable storage medium according to claim 9, wherein the surgical tool is coupled to a robotic arm of a surgical robot, and the instructions, when executed by the processor, further cause the computer to cause the surgical robot to reposition the surgical tool when it is determined that the light sensor coupled to the surgical tool is not detecting at least the predetermined amount of light emitted by the light source coupled to the image capture device; preferably wherein the surgical tool is repositioned towards the image capture device until the light sensor detects at least the predetermined amount of light emitted by the light source coupled to the image capture device.

11. The non-transitory computer-readable storage medium according to claim 9 or claim 10, wherein the surgical tool is a first surgical tool and the light sensor is a first light sensor, and the instructions, when executed by the processor, further cause the computer to:
determine whether a second light sensor coupled to a second surgical tool is detecting at least a second predetermined amount of light emitted by the light source coupled to the image capture device; and
provide a notification when it is determined that the second light sensor coupled to the second surgical tool is not detecting at least a second predetermined amount of light emitted by the light source coupled to the image capture device; preferably wherein the second surgical tool is coupled to a robotic arm of a surgical robot, and the instructions, when executed by the processor, further cause the computer to cause the surgical robot to reposition the second surgical tool when it is determined that the second light sensor coupled to the second surgical tool is not detecting at least the second predetermined amount of light emitted by the light source coupled to the image capture device.

12. The non-transitory computer-readable storage medium according to any of claims 9 to 11, wherein the instructions, when executed by the processor, further cause the computer to:
determine whether the surgical tool is inserted into a trocar;
open a door for the light sensor when it is determined that the surgical tool is inserted into the trocar; and
close the door for the light sensor when it is determined that the surgical tool is not inserted into the trocar; preferably when executed by the processor, further cause the computer to prevent activation of the surgical tool when it is determined that the light sensor coupled to the surgical tool is not detecting at least the predetermined amount of light emitted by the light source coupled to the image capture device.

13. The non-transitory computer-readable storage medium according to any of claims 9 to 12, wherein the instructions, when executed by the processor, further cause the computer to activate a pump to circulate coolant through the surgical tool when it is determined that the light sensor coupled to the surgical tool is not detecting at least the predetermined amount of light emitted by the light source coupled to the image capture device.

14. The non-transitory computer-readable storage medium according to any of claims 9 to 13, wherein the surgical tool is an electrosurgical tool, and the instructions, when executed by the processor, further cause the computer to prevent an electrosurgical generator from providing electrosurgical energy to the surgical tool when it is determined that the light sensor coupled to the surgical tool is not detecting at least the predetermined amount of light emitted by the light source coupled to the image capture device.

15. The non-transitory computer-readable storage medium according to any of claims 9 to 14, wherein the predetermined amount of light is less than a total amount of light emitted by the light source coupled to the image capture device.

## Patentansprüche

1. System (100) für eine Roboterchirurgie, das System umfassend:
einen chirurgischen Roboter, der eine Vielzahl von Roboterarmen (115a, 115b) einschließt;
eine Bilderfassungsvorrichtung (130) und eine Lichtquelle (137), die beide mit einem ersten der Vielzahl von Roboterarmen wirkgekoppelt sind, ein chirurgisches Werkzeug (120), das mit einem zweiten der Vielzahl von Roboterarmen wirkgekoppelt ist, und einen Lichtsensor (127), der sich an dem distalen Ende des Werkzeugs befindet; und
eine Rechenvorrichtung (200), die einen Prozessor (204) und einen Speicher (202) einschließt, die Anweisungen speichern, die,
wenn sie durch den Prozessor ausgeführt werden, die Computervorrichtung veranlassen zum:
Bestimmen, ob der Lichtsensor, der mit dem chirurgischen Werkzeug gekoppelt ist, mindestens eine zuvor bestimmte Lichtmenge erkennt, die durch die Lichtquelle emittiert wird, die mit der Bilderfassungsvorrichtung gekoppelt ist; und
Bereitstellen einer Benachrichtigung, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

2. System nach Anspruch 1, wobei die Anweisungen der Computervorrichtung, wenn sie durch den Prozessor ausgeführt werden, die Computervorrichtung ferner veranlassen, den chirurgischen Roboter zu veranlassen, das chirurgische Werkzeug neu zu positionieren, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

3. System nach Anspruch 2, wobei das chirurgische Werkzeug zu der Bilderfassungsvorrichtung hin neu positioniert wird, bis der Lichtsensor mindestens die zuvor bestimmte Lichtmenge erkennt, die durch die Lichtquelle emittiert wird, die mit der Bilderfassungsvorrichtung gekoppelt ist.

4. System nach Anspruch 2, wobei das chirurgische Werkzeug ein erstes chirurgisches Werkzeug ist und der Lichtsensor ein erster Lichtsensor ist, das System ferner umfassend:
ein zweites chirurgisches Werkzeug, das mit einem dritten der Vielzahl von Roboterarmen gekoppelt ist, und einen zweiten Lichtsensor,
wobei die Anweisungen, wenn sie durch den Prozessor ausgeführt werden, ferner die Computervorrichtung veranlassen zum:
Bestimmen, ob der zweite Lichtsensor, der mit dem zweiten chirurgischen Werkzeug gekoppelt ist, mindestens eine zweite zuvor bestimmte Lichtmenge erkennt, die durch die Lichtquelle emittiert wird, die mit der Bilderfassungsvorrichtung gekoppelt ist; und
Bereitstellen einer Benachrichtigung, wenn bestimmt wird, dass der zweite Lichtsensor, der mit dem zweiten chirurgischen Werkzeug gekoppelt ist, nicht mindestens die zweite zuvor bestimmte Lichtmenge erkennt, die durch die Lichtquelle emittiert wird, die mit der Bilderfassungsvorrichtung gekoppelt ist; vorzugsweise wobei die Anweisungen, wenn sie durch den Prozessor ausgeführt werden, die Computervorrichtung ferner veranlassen, den chirurgischen Roboter zu veranlassen, das zweite chirurgische Werkzeug neu zu positionieren, wenn bestimmt wird, dass der zweite Lichtsensor, der mit dem zweiten chirurgischen Werkzeug gekoppelt ist, nicht mindestens die zweite zuvor bestimmte Lichtmenge erkennt, die durch die Lichtquelle emittiert wird, die mit der Bilderfassungsvorrichtung gekoppelt ist.

5. System nach einem der vorstehenden Ansprüche, wobei sich der Lichtsensor, der mit dem chirurgischen Werkzeug gekoppelt ist, an einem distalen Abschnitt des chirurgischen Werkzeugs befindet; und/oder ferner umfassend eine Tür für den Lichtsensor, der mit dem chirurgischen Werkzeug gekoppelt ist,
wobei die Anweisungen, wenn sie durch den Prozessor ausgeführt werden, ferner die Computervorrichtung veranlassen zum:
Bestimmen, ob das chirurgische Werkzeug in einen Trokar eingeführt ist;
Veranlassen des chirurgischen Roboters, die Tür für den Lichtsensor zu öffnen, wenn bestimmt wird, dass das chirurgische Werkzeug in den Trokar eingeführt ist; und
Veranlassen des chirurgischen Roboters, die Tür für den Lichtsensor zu schließen, wenn bestimmt wird, dass das chirurgische Instrument nicht in den Trokar eingeführt ist.

6. System nach einem der vorstehenden Ansprüche, wobei die Anweisungen, wenn sie durch den Prozessor ausgeführt werden, ferner die Computervorrichtung veranlassen, eine Aktivierung des chirurgischen Werkzeugs zu verhindern, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist; und/oder ferner umfassend:
eine Pumpe zum Zirkulieren von Kühlmittel durch das chirurgische Werkzeug hindurch,
wobei die Anweisungen die Computervorrichtung ferner veranlassen, die Pumpe zu aktivieren, um Kühlmittel durch das chirurgische Werkzeug hindurch zu zirkulieren, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

7. System nach einem der vorstehenden Ansprüche, wobei das chirurgische Werkzeug ein elektrochirurgisches Werkzeug ist, und das System ferner umfasst:
einen elektrochirurgischen Generator, der konfiguriert ist, um elektrochirurgische Energie an das chirurgische Werkzeug bereitzustellen,
wobei die Anweisungen, wenn sie durch den Prozessor ausgeführt werden, ferner die Computervorrichtung veranlassen, den elektrochirurgischen Generator daran zu hindern, dem chirurgischen Werkzeug elektrochirurgische Energie bereitzustellen, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

8. System nach einem der vorstehenden Ansprüche, wobei die Bilderfassungsvorrichtung ein Laparoskop ist; und/oder wobei die zuvor bestimmte Lichtmenge geringer als eine Gesamtlichtmenge ist, die durch die Lichtquelle emittiert wird, die mit der Bilderfassungsvorrichtung gekoppelt ist.

9. Nichtflüchtiges, computerlesbares Speichermedium, das Anweisungen speichert, die, wenn sie durch einen Prozessor (204) in einem System für Roboterchirurgie ausgeführt werden, das System (100) umfassend:
einen chirurgischen Roboter, der eine Vielzahl von Roboterarmen (115, 115b) einschließt:
eine Bilderfassungsvorrichtung (130) und eine Lichtquelle (137), die beide mit einem ersten der Vielzahl von Roboterarmen wirkgekoppelt sind und
ein chirurgisches Werkzeug (120), das mit einem zweiten der Vielzahl von Roboterarmen wirkgekoppelt ist, und einen Lichtsensor (127), der sich an dem distalen Ende des Werkzeugs befindet:
einen Computer (200) veranlassen zum:
Bestimmen, ob ein Lichtsensor, der mit dem chirurgischen Werkzeug gekoppelt ist, mindestens eine zuvor bestimmte Lichtmenge erkennt, die durch die Lichtquelle emittiert wird, die mit einer Bilderfassungsvorrichtung gekoppelt ist; und
Bereitstellen einer Benachrichtigung, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

10. Nichtflüchtiges, computerlesbare Speichermedium nach Anspruch 9, wobei das chirurgische Werkzeug mit einem Roboterarm eines chirurgischen Roboters gekoppelt ist und die Anweisungen, wenn sie durch den Prozessor ausgeführt werden, den Computer ferner veranlassen, den chirurgischen Roboter zu veranlassen, das chirurgische Werkzeug neu zu positionieren, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist; vorzugsweise wobei das chirurgische Werkzeug zu der Bilderfassungsvorrichtung hin neu positioniert wird, bis der Lichtsensor mindestens die zuvor bestimmte Lichtmenge erkennt, die durch die Lichtquelle emittiert wird, die mit der Bilderfassungsvorrichtung gekoppelt ist.

11. Nichtflüchtiges computerlesbares Speichermedium nach Anspruch 9 oder 10, wobei das chirurgische Werkzeug ein erstes chirurgisches Werkzeug und der Lichtsensor ein erster Lichtsensor ist, und die Anweisungen, wenn sie durch den Prozessor ausgeführt werden, den Computer ferner veranlassen zum:
Bestimmen, ob ein zweiter Lichtsensor, der mit einem zweiten chirurgischen Werkzeug gekoppelt ist, mindestens eine zweite zuvor bestimmte Lichtmenge erkennt, die durch die Lichtquelle emittiert wird, die mit der Bilderfassungsvorrichtung gekoppelt ist; und
Bereitstellen einer Benachrichtigung, wenn bestimmt wird, dass der zweite Lichtsensor, der mit dem zweiten chirurgischen Werkzeug gekoppelt ist, nicht mindestens eine zweite zuvor bestimmte Lichtmenge erkennt, die durch die Lichtquelle emittiert wird, die mit der Bilderfassungsvorrichtung gekoppelt ist; vorzugsweise wobei das zweite chirurgische Werkzeug mit einem Roboterarm eines chirurgischen Roboters gekoppelt ist und die Anweisungen, wenn sie durch den Prozessor ausgeführt werden, den Computer ferner veranlassen, den chirurgischen Roboter zu veranlassen, das zweite chirurgische Werkzeug neu zu positionieren, wenn bestimmt wird, dass der zweite Lichtsensor, der mit dem zweiten chirurgischen Werkzeug gekoppelt ist, nicht mindestens die zweite zuvor bestimmte Lichtmenge erkennt, die durch die Lichtquelle emittiert wird, die mit der Bilderfassungsvorrichtung gekoppelt ist.

12. Nicht flüchtiges computerlesbares Speichermedium nach einem der Ansprüche 9 bis 11, wobei die Anweisungen, wenn sie durch den Prozessor ausgeführt werden, den Prozessor ferner veranlassen zum:
Bestimmen, ob das chirurgische Werkzeug in einen Trokar eingeführt ist;
Öffnen einer Tür für den Lichtsensor, wenn bestimmt wird, dass das chirurgische Werkzeug in den Trokar eingeführt ist; und
Schließen der Tür für den Lichtsensor, wenn bestimmt wird, dass das chirurgische Werkzeug nicht in den Trokar eingeführt ist; vorzugsweise wenn es durch den Prozessor ausgeführt wird, ferner Veranlassen des Computers, die Aktivierung des chirurgischen Werkzeugs zu verhindern, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

13. Nichtflüchtiges computerlesbares Speichermedium nach einem der Ansprüche 9 bis 12, wobei die Anweisungen, wenn sie durch den Prozessor ausgeführt werden, den Computer ferner veranlassen, eine Pumpe zu aktivieren, um Kühlmittel durch das chirurgische Werkzeug hindurch zu zirkulieren, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

14. Nichtflüchtiges computerlesbares Speichermedium nach einem der Ansprüche 9 bis 13, wobei das chirurgische Werkzeug ein elektrochirurgisches Werkzeug ist und die Anweisungen, wenn sie durch den Prozessor ausgeführt werden, den Computer ferner veranlassen, einen elektrochirurgischen Generator daran zu hindern, elektrochirurgische Energie an das chirurgische Werkzeug bereitzustellen, wenn bestimmt wird, dass der Lichtsensor, der mit dem chirurgischen Werkzeug gekoppelt ist, nicht mindestens die zuvor bestimmte Lichtmenge erkennt, die durch die Lichtquelle emittiert wird, die mit der Bilderfassungsvorrichtung gekoppelt ist.

15. Nichtflüchtiges computerlesbares Speichermedium nach einem der Ansprüche 9 bis 14, wobei die zuvor bestimmte Lichtmenge geringer als eine Gesamtlichtmenge ist, die durch die Lichtquelle emittiert wird, die mit der Bilderfassungsvorrichtung gekoppelt ist.

## Revendications

1. Système (100) de chirurgie robotique, le système comprenant :
un robot chirurgical comportant une pluralité de bras robotiques (115a, 115b) ;
un dispositif de capture d'images (130) et une source de lumière (137) tous deux couplés de manière opérationnelle à un premier de la pluralité de bras robotiques, un outil chirurgical (120) couplé de manière opérationnelle à un deuxième de la pluralité de bras robotiques et un capteur de lumière (127) situé au niveau de l'extrémité distale dudit outil ; et
un dispositif informatique (200) comportant un processeur (204) et une mémoire (202) stockant des instructions qui,
lorsqu'elles sont exécutées par le processeur, amènent le dispositif informatique à :
déterminer si le capteur de lumière couplé à l'outil chirurgical détecte au moins une quantité prédéterminée de lumière émise par la source de lumière couplée au dispositif de capture d'images ; et
fournir une notification lorsqu'il est déterminé que le capteur de lumière couplé à l'outil chirurgical ne détecte pas au moins la quantité prédéterminée de lumière émise par la source de lumière couplée au dispositif de capture d'images.

2. Système selon la revendication 1, dans lequel les instructions du dispositif informatique, lorsqu'elles sont exécutées par le processeur, amènent en outre le dispositif informatique à amener le robot chirurgical à repositionner l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images.

3. Système selon la revendication 2, dans lequel l'outil chirurgical est repositionné vers le dispositif de capture d'images jusqu'à ce que le capteur de lumière détecte au moins la quantité prédéterminée de lumière émise par la source de lumière couplée au dispositif de capture d'images.

4. Système selon la revendication 2, dans lequel l'outil chirurgical est un premier outil chirurgical et le capteur de lumière est un premier capteur de lumière, le système comprenant en outre :
un second outil chirurgical couplé à un troisième de la pluralité de bras robotiques et à un second capteur de lumière,
dans lequel les instructions, lorsqu'elles sont exécutées par le processeur, amènent en outre le dispositif informatique à :
déterminer si le second capteur de lumière couplé au second outil chirurgical détecte au moins une seconde quantité prédéterminée de lumière émise par la source de lumière couplée au dispositif de capture d'images ; et
fournir une notification lorsqu'il est déterminé que le second capteur de lumière couplé au second outil chirurgical ne détecte pas au moins la seconde quantité prédéterminée de lumière émise par la source de lumière couplée au dispositif de capture d'images ; de préférence, dans lequel les instructions, lorsqu'elles sont exécutées par le processeur, amènent en outre le dispositif informatique à amener le robot chirurgical à repositionner le second outil chirurgical lorsqu'il est déterminé que le second capteur de lumière couplé au second outil chirurgical ne détecte pas au moins la seconde quantité prédéterminée de lumière émise par la source de lumière couplée au dispositif de capture d'images.

5. Système selon l'une quelconque revendication précédente, dans lequel le capteur de lumière couplé à l'outil chirurgical est situé au niveau d'une partie distale de l'outil chirurgical ; et/ou comprenant en outre une porte pour le capteur de lumière couplé à l'outil chirurgical,
dans lequel les instructions, lorsqu'elles sont exécutées par le processeur, amènent en outre le dispositif informatique à :
déterminer si l'outil chirurgical est inséré dans un trocart ;
amener le robot chirurgical à ouvrir la porte pour le capteur de lumière lorsqu'il est déterminé que l'outil chirurgical est inséré dans le trocart ; et
amener le robot chirurgical à fermer la porte pour le capteur de lumière lorsqu'il est déterminé que l'outil chirurgical n'est pas inséré dans le trocart.

6. Système selon l'une quelconque revendication précédente, dans lequel les instructions, lorsqu'elles sont exécutées par le processeur, amènent en outre le dispositif informatique à empêcher l'activation de l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images ; et/ou comprenant en outre :
une pompe pour faire circuler le liquide de refroidissement à travers l'outil chirurgical,
dans lequel les instructions amènent en outre le dispositif informatique à activer la pompe pour faire circuler le liquide de refroidissement à travers l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images.

7. Système selon l'une quelconque revendication précédente, dans lequel l'outil chirurgical est un outil électrochirurgical, et le système comprend en outre :
un générateur électrochirurgical configuré pour fournir de l'énergie électrochirurgicale à l'outil chirurgical,
dans lequel les instructions, lorsqu'elles sont exécutées par le processeur, amènent en outre le dispositif informatique à empêcher le générateur électrochirurgical de fournir de l'énergie électrochirurgicale à l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images.

8. Système selon l'une quelconque revendication précédente, dans lequel le dispositif de capture d'images est un laparoscope ; et/ou dans lequel la quantité de lumière prédéterminée est inférieure à une quantité totale de lumière émise par la source de lumière couplée au dispositif de capture d'images.

9. Support de stockage non transitoire lisible par ordinateur stockant des instructions qui, lorsqu'elles sont exécutées par un processeur (204) dans un système de chirurgie robotique, le système (100) comprenant :
un robot chirurgical comportant une pluralité de bras robotiques (115, 115b) :
un dispositif de capture d'images (130) et une source de lumière (137) tous deux couplés de manière opérationnelle à un premier de la pluralité de bras robotiques et
un outil chirurgical (120) couplé de manière opérationnelle à un deuxième de la pluralité de bras robotiques et à un capteur de lumière (127) situé au niveau de l'extrémité distale dudit outil :
amener un ordinateur (200) à :
déterminer si un capteur de lumière couplé à l'outil chirurgical détecte au moins une quantité prédéterminée de lumière émise par la source de lumière couplée à un dispositif de capture d'images ; et
fournir une notification lorsqu'il est déterminé que le capteur de lumière couplé à l'outil chirurgical ne détecte pas au moins la quantité prédéterminée de lumière émise par la source de lumière couplée au dispositif de capture d'images.

10. Support de stockage non transitoire lisible par ordinateur selon la revendication 9, dans lequel l'outil chirurgical est couplé à un bras robotique d'un robot chirurgical, et les instructions, lorsqu'elles sont exécutées par le processeur, amènent en outre l'ordinateur à amener le robot chirurgical à repositionner l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images ; de préférence, dans lequel l'outil chirurgical est repositionné vers le dispositif de capture d'images jusqu'à ce que le capteur de lumière détecte au moins la quantité prédéterminée de lumière émise par la source de lumière couplée au dispositif de capture d'images.

11. Support de stockage non transitoire lisible par ordinateur selon la revendication 9 ou la revendication 10, dans lequel l'outil chirurgical est un premier outil chirurgical et le capteur de lumière est un premier capteur de lumière, et les instructions, lorsqu'elles sont exécutées par le processeur, amènent en outre l'ordinateur à :
déterminer si un second capteur de lumière couplé à un second outil chirurgical détecte au moins une seconde quantité prédéterminée de lumière émise par la source de lumière couplée au dispositif de capture d'images ; et
fournir une notification lorsqu'il est déterminé que le second capteur de lumière couplé au second outil chirurgical ne détecte pas au moins une seconde quantité prédéterminée de lumière émise par la source de lumière couplée au dispositif de capture d'images ; de préférence, dans lequel le second outil chirurgical est couplé à un bras robotique d'un robot chirurgical, et les instructions, lorsqu'elles sont exécutées par le processeur, amènent en outre l'ordinateur à amener le robot chirurgical à repositionner le second outil chirurgical lorsqu'il est déterminé que le second capteur de lumière couplé au second outil chirurgical ne détecte pas au moins la seconde quantité prédéterminée de lumière émise par la source de lumière couplée au dispositif de capture d'images.

12. Support de stockage non transitoire lisible par ordinateur selon l'une quelconque des revendications 9 à 11, dans lequel les instructions, lorsqu'elles sont exécutées par le processeur, amènent en outre le processeur à :
déterminer si l'outil chirurgical est inséré dans un trocart ;
ouvrir une porte pour le capteur de lumière lorsqu'il est déterminé que l'outil chirurgical est inséré dans le trocart ; et
fermer la porte pour le capteur de lumière lorsqu'il est déterminé que l'outil chirurgical n'est pas inséré dans le trocart ; de préférence, lorsqu'elles sont exécutées par le processeur, amènent en outre l'ordinateur à empêcher l'activation de l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images.

13. Support de stockage non transitoire lisible par ordinateur selon l'une quelconque des revendications 9 à 12, dans lequel les instructions, lorsqu'elles sont exécutées par le processeur, amènent en outre l'ordinateur à activer une pompe pour faire circuler un liquide de refroidissement à travers l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images.

14. Support de stockage non transitoire lisible par ordinateur selon l'une quelconque des revendications 9 à 13, dans lequel l'outil chirurgical est un outil électrochirurgical, et les instructions, lorsqu'elles sont exécutées par le processeur, amènent en outre l'ordinateur à empêcher un générateur électrochirurgical de fournir de l'énergie électrochirurgicale à l'outil chirurgical lorsqu'il est déterminé que le capteur de lumière couplé à l'outil chirurgical ne détecte pas au moins la quantité prédéterminée de lumière émise par la source de lumière couplée au dispositif de capture d'images.

15. Support de stockage non transitoire lisible par ordinateur selon l'une quelconque des revendications 9 à 14, dans lequel la quantité prédéterminée de lumière est inférieure à la quantité totale de lumière émise par la source de lumière couplée au dispositif de capture d'images.
